# EUROPEAN PATENT SPECIFICATION

(11) **EP 4 045 063 B1**
(45) Date of publication and mention of the grant of the patent: **02.10.2024**
(21) Application number: 19957323.9
(22) Date of filing: 27.12.2019
(51) Int. Cl.: A61K 33/242, A61K 47/64, A61K 47/54, A61P 25/00, A61P 37/02, A61K 47/62, A61K 47/69, A61P 25/28, B82Y 5/00

(54) **COMPOSITION AND METHOD FOR TREATMENT OF MULTIPLE SCLEROSIS**
GOLD CLUSTER (AUC) ZUR BEHANDLUNG VON MULTIPLER SKLEROSE
AGRÉGATS D'OR (AUC) POUR LE TRAITEMENT DE LA SCLEROSE EN PLAQUES

(43) Date of publication of application: 24.08.2022
(73) Proprietor: Wuhan Vast Conduct Science Foundation Co., Ltd., Wuhan, Hubei 430070 (CN)
(72) Inventor: SUN, Taolei, Wuhan, Hubei 430070 (CN)
(74) Representative: Meyer, Thorsten
(86) International application number: PCT/CN2019/129264
(87) International publication number: WO 2021/128292

(56) References cited:
- WO-A1-2018/024111
- CN-A- 1 939 332
- CN-A- 111 035 653
- CN-A- 111 035 653
- US-A1- 2014 105 980
- US-A1- 2016 015 742
- US-A1- 2019 030 069
- US-A1- 2019 175 753
- ASSUNTA DAL BIANCO ET AL: "Multiple sclerosis and Alzheimer's disease", ANNALS OF NEUROLOGY, JOHN WILEY AND SONS, BOSTON , US, vol. 63, no. 2, 9 October 2007 (2007-10-09), pages 174 - 183, XP071638401, ISSN: 0364-5134, DOI: 10.1002/ANA.21240
- DAVID MONIQUE ANTOINETTE ET AL: "Detection of Protein Aggregates in Brain and Cerebrospinal Fluid Derived from Multiple Sclerosis Patients", FRONTIERS IN NEUROLOGY, vol. 5, 2 December 2014 (2014-12-02), XP055823948, Retrieved from the Internet <URL:https://www.ncbi.nlm.nih.gov/pmc/articles/PMC4252634/pdf/fneur-05-00251.pdf> DOI: 10.3389/fneur.2014.00251
- ANONYMOUS: "Physiological Effects of N-Acetyl Cysteine in Patients With Multiple Sclerosis - NCT03032601", CLINICALTRIALS.GOV, 26 January 2017 (2017-01-26), Internet, pages 1 - 6, XP055964063, Retrieved from the Internet <URL:https://clinicaltrials.gov/ct2/show/record/NCT03032601> [retrieved on 20220923]
- GUANBIN GAO, RUI CHEN, MENG HE, JING LI, LIYUN WANG, TAOLEI SUN: "Gold nanoclusters for Parkinson's disease treatment", BIOMATERIALS, ELSEVIER, AMSTERDAM, NL, vol. 194, 1 February 2019 (2019-02-01), AMSTERDAM, NL, pages 36 - 46, XP055689788, ISSN: 0142-9612, DOI: 10.1016/j.biomaterials.2018.12.013
- ZHANG WENKANG, GAO GUANBIN, MA ZHONGJIE, LUO ZHUOYING, HE MENG, SUN TAOLEI: "Au23(CR)14 nanocluster restores fibril Aβ’s unfolded state with abolished cytotoxicity and dissolves endogenous Aβ plaques.", NATIONAL SCIENCE REVIEW, vol. 7, no. 4, 1 April 2020 (2020-04-01), pages 763 - 774, XP055823946, ISSN: 2095-5138, DOI: 10.1093/nsr/nwz215
- DAVID MONIQUE ANTOINETTE, TAYEBI MOURAD: "Detection of Protein Aggregates in Brain and Cerebrospinal Fluid Derived from Multiple Sclerosis Patients", FRONTIERS IN NEUROLOGY, vol. 5, 2 December 2014 (2014-12-02), XP055823948, DOI: 10.3389/fneur.2014.00251
- CHANDRA AVINASH.: "Role of Amyloid from a Multiple Sclerosis Perspective: A Literature Review.", NEUROIMMUNOMODULATION., vol. 22, no. 6, 1 November 2015 (2015-11-01), pages 1 - 4, XP009528655, ISSN: 1021-7401, DOI: 10.1159/000375309
- DOLATI SANAM; BABALOO ZOHREH; JADIDI-NIARAGH FARHAD; AYROMLOU HORMOZ; SADREDDINI SANAM; YOUSEFI MEHDI: "Multiple sclerosis: Therapeutic applications of advancing drug delivery systems", BIOMEDICINE AND PHARMACOTHERAPY., ELSEVIER, FR, vol. 86, 21 December 2016 (2016-12-21), FR, pages 343 - 353, XP029884769, ISSN: 0753-3322, DOI: 10.1016/j.biopha.2016.12.010
- GONG DEJUN : "Chiral Gold Nanoclusters: Synthesis, Properties and Applications.", PROGRESS IN CHEMISTRY., vol. 28, no. 2-3, 15 March 2016 (2016-03-15), pages 296 - 307, XP009528654, ISSN: 1005-281X, DOI: 10.7536/PC150931

## Description

### FIELD OF THE DISCLOSURE

The present disclosure relates to gold cluster (AuC) for use in the treatment of multiple sclerosis.

### BACKGROUND OF THE DISCLOSURE

Multiple sclerosis (MS), meaning "scar tissue in multiple areas", is an autoimmune disorder in which the immune system attacks the myelin sheath that surrounds and protects the nerve fibers of the central nervous system (CNS), causing inflammation. When myelin or nerve fibers are damaged or destroyed in MS, damage to areas of the CNS may produce a variety of neurological symptoms that vary among people with MS in type and severity. There are four types of MS: clinically isolated syndrome (CIS), relapse-remitting MS (RRMS), primary progressive MS (PPMS), and secondary progressive MS (SPMS). The common symptoms include muscle weakness, numbness and tingling, Lhermitte's sign, bladder problems, bowel problems, fatigue, dizziness and vertigo, sexual dysfunction, spasticity and muscle spasms, tremor, vision problems, gait and mobility changes, emotional changes and depression, learning and memory problems, and pain.

The cause of MS is not known, but it is believed to involve genetic susceptibility, abnormalities in the immune system and environmental factors that combine to trigger the disease.

While available medications are helpful to slow progression by changing the way the immune system functions or relieve symptoms during a flare when a person experiences a worsening of symptoms, there is still an imperative need for new medications and methods for the treatment of MS.

WO 2018/024111 A1 discloses a composition comprising gold clusters which is used to prepare drugs for preventing and treating Alzheimer's disease and/or Parkinson's disease (PD). It does not involve the pharmaceutical application of the gold clusters composition in other diseases, such as an autoimmune disease.

Bianco A, *et al.* involve the study of microglia activation in MS patients and its influence on the development of AD lesions. The disclosure fails to reveal any direct relation between MS syndrome and development of AD pathology. Aβ plaques was also investigated in the MS cases, where no difference in the density of Aβ plaques between the demyelinated and nondemyelinated area of the MS cortex was observed. The disclosure teaches away from the treatment of MS targeting to Aβ plaques.

David A, *et al.* disclose that Amyloid β, tau protein, and amyloid precursor protein associated with AD and PD (Parkinson's disease) are found in lesions and plaques of MS patients. But it does not confirm the role Amyloid β plays in MS pathogenesis, let alone in prevention or treatment of MS.

The previous report (Anonymous: "Physiological Effects of N-Acetyl Cysteine in Patients With Multiple Sclerosis-NCT03032601" Clinical Trials.gov, 26 January 2017, pages 1-6, XP055964063, URL: https://clinicaltrails.gov/ct2/show/record/NCT03032601) discloses a clinical trial regarding to treatment of MS. But it does not involve pharmaceutical use of gold clusters or inhibition of Aβ plaques in MS.

CN 111035653 A is a corresponding application of the present disclosure filed to CNIPA on the same application date.

### SUMMARY OF THE DISCLOSURE

The objective of the present disclosure is to provide a gold cluster (AuC) for use in the treatment of multiple sclerosis, the AuC comprises a gold core; and a ligand bonded the gold core;
the ligand is one selected from the group consisting of L-cysteine and its derivatives, D-cysteine and its derivatives, and cysteine-containing oligopeptides and their derivatives;
the L-cysteine and its derivatives are selected from the group consisting of L-cysteine, N-isobutyryl-L-cysteine (L-NIBC), and N-acetyl-L-cysteine (L-NAC); and the D-cysteine and its derivatives are selected from the group consisting of D-cysteine, N-isobutyryl D-cysteine (D-NIBC), and N-acetyl-D-cysteine (D-NAC);
the cysteine-containing oligopeptides and their derivatives are cysteine-containing dipeptides, cysteine-containing tripeptides or cysteine-containing tetrapeptides;
the cysteine-containing dipeptides are selected from the group consisting of L-cysteine-L-arginine dipeptide (CR), L-arginine-L-cysteine dipeptide (RC), L-histidine-L-cysteine dipeptide (HC), and L-cysteine-L-histidine dipeptide (CH);
the cysteine-containing tripeptides are selected from the group consisting of glycine L-cysteine-L-arginine tripeptide (GCR), L-proline-L-cysteine-L-arginine tripeptide (PCR), L-lysine-L-cysteine-L-proline tripeptide (KCP), and L-glutathione (GSH); and
the cysteine-containing tetrapeptides are selected from the group consisting of glycine-L-serine-L-cysteine-L-arginine tetrapeptide (GSCR), and glycine-L-cysteine-L-serine-L-arginine tetrapeptide (GCSR).

In certain embodiments of the AuC for use, the gold core has a diameter smaller than 3 nm.

In certain embodiments, the gold core has a diameter in the range of 0.5-2.6 nm.

The objectives and advantages of the disclosure will become apparent from the following detailed description of preferred embodiments thereof in connection with the accompanying drawings.

### Description of the Drawings

Preferred embodiments according to the present disclosure will now be described with reference to the Figures, in which like reference numerals denote like elements.
FIG 1 shows ultraviolet-visible (UV) spectrums, transmission electron microscope (TEM) images and particle size distribution diagrams of ligand L-NIBC-modified gold nanoparticles (L-NIBC-AuNPs) with different particle sizes.
FIG 2 shows ultraviolet-visible (UV) spectrums, TEM images and particle size distribution diagrams of ligand L-NIBC-bonded gold clusters (L-NIBC-AuCs) with different particle sizes.
FIG 3 shows infrared spectra of L-NIBC-AuCs with different particle sizes.
FIG 4 shows UV, infrared, TEM, and particle size distribution diagrams of ligand CR-bonded gold clusters (CR-AuCs).
FIG 5 shows UV, infrared, TEM, and particle size distribution diagrams of ligand RC-bonded gold clusters (RC-AuCs).
FIG 6 shows UV, infrared, TEM, and particle size distribution diagrams of ligand 1-[(2S)-2-methyl-3-thiol-1-oxopropyl]-L-proline (i.e., Cap)-bonded gold clusters (Cap-AuCs).
FIG 7 shows UV, infrared, TEM, and particle size distribution diagrams of ligand GSH-bonded gold clusters (GSH-AuCs).
FIG 8 shows UV, infrared, TEM, and particle size distribution diagrams of ligand D-NIBC-bonded gold clusters (D-NIBC-AuCs).
FIG 9 shows UV, infrared, TEM, and particle size distribution diagrams of ligand L-cysteine-bonded gold clusters (L-Cys-AuCs).
FIG 10 is a graph showing the clinical scores of EAE.
FIG 11 shows the levels of (a) TNF-α, (b) IL-17 and (c) IFN-γ in spinal cords, wherein 1 denotes for naive group, 2 for saline-treated group, 3 for prednisone-treated group, 4 for 50mg/kg L-Cys-AuCs-treated group, 5 for 20mg/kg L-Cys-AuCs-treated group, and 6 for 5mg/kg L-Cys-AuCs-treated group.
FIG 12 shows (a) pictures of HE staining for spinal cord showing the infiltration of immune cells into the spinal cord, and (b) a bar graph showing histological scales of inflammation; wherein 1 denotes for naive group, 2 for saline-treated group, 3 for prednisone-treated group, 4 for 50mg/kg L-Cys-AuCs-treated group, 5 for 20mg/kg L-Cys-AuCs-treated group, and 6 for 5mg/kg L-Cys-AuCs-treated group.

### Detailed Description of the Embodiments

The present disclosure may be understood more readily by reference to the following detailed description of certain embodiments of the disclosure. The following examples or embodiments which are not part of the scope of the claims are present for illustration purposes only.

Gold clusters (AuCs) are a special form of gold existing between gold atoms and gold nanoparticles. AuCs have a size smaller than 3 nm, and are composed of only several to a few hundreds of gold atoms, leading to the collapse of face-centered cubic stacking structure of gold nanoparticles. As a result, AuCs exhibit molecule-like discrete electronic structures with distinct HOMO-LUMO gap unlike the continuous or quasi-continuous energy levels of gold nanoparticles. This leads to the disappearance of surface plasmon resonance effect and the corresponding plasmon resonance absorption band (520 ± 20 nm) at uv-vis spectrum that possessed by conventional gold nanoparticles.

The present disclosure provides a ligand-bonded AuC.

In certain embodiments, the ligand-bonded AuC comprises a ligand and a gold core, wherein the ligand is bonded to the gold core. In certain embodiments, the diameter of the gold core is in the range of 0.5 - 3 nm. In certain embodiments, the diameter of the gold core is in the range of 0.5 - 2.6 nm.

In certain embodiments, the ligand of the ligand-bonded AuC is a thiol-containing compound or oligopeptide. In certain embodiments, the ligand bonds to the gold core to form a ligand-bonded AuC via Au-S bond.

In certain embodiments, the ligand is one selected from the group consisting of L-cysteine, D-cysteine, and a cysteine derivative. In certain embodiments, the cysteine derivative is one selected from the group consisting of N-isobutyryl-L-cysteine (L-NIBC), N-isobutyryl-D-cysteine (D-NIBC), N-acetyl-L-cysteine (L-NAC), and N-acetyl-D-cysteine (D-NAC).

In certain embodiments, the ligand is one selected from the group consisting of a cysteine-containing oligopeptide and its derivatives. In certain embodiments, the cysteine-containing oligopeptide is a cysteine-containing dipeptide. In certain embodiments, the cysteine-containing dipeptide is one selected from the group consisting of L-cysteine-L-arginine dipeptide (CR), L-arginine-L-cysteine dipeptide (RC), L-histidine-L-cysteine dipeptide (HC) and L-cysteine-L-histidine dipeptide (CH). In certain embodiments, the cysteine-containing oligopeptide is a cysteine-containing tripeptide. In certain embodiments, the cysteine-containing tripeptide is one selected from the group consisting of glycine-L-cysteine-L-arginine tripeptide (GCR), L-proline-L-cysteine-L-arginine tripeptide (PCR), L-lysine-L-cysteine-L-proline tripeptide (KCP), and L-glutathione (GSH). In certain embodiments, the cysteine-containing oligopeptide is a cysteine-containing tetrapeptide. In certain embodiments, the cysteine-containing tetrapeptide is one selected from the group consisting of glycine-L-serine-L-cysteine-L-arginine tetrapeptide (GSCR) and glycine-L-cysteine-L-serine-L-arginine tetrapeptide (GCSR).

According to some embodiments not part of the scope of the claims, the ligand is a thiol-containing compound. According to some embodiments not part of the scope of the claims, thiol-containing compound is one selected from the group consisting of 1-[(2S)-2-methyl-3-thiol-1-oxopropyl]-L-proline, thioglycollic acid, mercaptoethanol, thiophenol, D-3-trolovol, N-(2-mercaptopropionyl)-glycine and dodecyl mercaptan.

The present disclosure provides a pharmaceutical composition for the treatment of multiple sclerosis in a subject. In certain embodiments, the subject is human. In certain embodiments, the subject is a pet animal such as a dog.

In certain embodiments, the pharmaceutical composition comprises a ligand-bonded AuC as disclosed above and a pharmaceutically acceptable excipient. In certain embodiments, the excipient is phosphate-buffered solution, or physiological saline.

The present disclosure provides a use of the above disclosed ligand-bonded AuCs for manufacturing a medication for the treatment of multiple sclerosis in a subject.

The present disclosure provides the above disclosed ligand-bonded AuCs for use in the treatment of multiple sclerosis in a subject using the above disclosed ligand-bonded AuCs. According to some embodiments not part of the scope of the claims, the treatment comprises administering a pharmaceutically effective amount of ligand-bonded AuCs to the subject. The pharmaceutically effective amount can be ascertained by routine *in vivo* studies.

The following examples are provided for the sole purpose of illustrating the principles of the present disclosure; they are by no means intended to limit the scope of the present disclosure.

### Examples

### 1. Preparation of ligand-bonded AuCs

1.1 Dissolving HAuCl₄ in methanol, water, ethanol, n-propanol, or ethyl acetate to get a solution A in which the concentration of HAuCl₄ is 0.01∼0.03M;
1.2 Dissolving a ligand in a solvent to get a solution B in which the concentration of the ligand is 0.01∼0.18M; the ligand includes, but not limited to, L-cysteine, D-cysteine and other cysteine derivatives such as N-isobutyryl-L-cysteine (L-NIBC), N-isobutyryl-D-cysteine (D-NIBC), N-acetyl-L-cysteine (L-NAC), and N-acetyl-D-cysteine (D-NAC), cysteine-containing oligopeptides and their derivatives including, but not limited to, dipeptides, tripeptide, tetrapeptide and other peptides containing cysteine, such as L-cysteine-L-arginine dipeptide (CR), L-arginine-L-cysteine dipeptide (RC), L-cysteine L-histidine (CH), glycine-L-cysteine-L-arginine tripeptide (GCR), L-proline-L-cysteine-L-arginine tripeptide (PCR), L-glutathione (GSH), glycine-L-serine-L-cysteine-L-arginine tetrapeptide (GSCR) and glycine-L-cysteine-L-serine-L-arginine tetrapeptide (GCSR), and other thiol-containing compounds, such as one or more of 1-[(2S)-2-methyl-3-thiol-1-oxopropyl]-L-proline, thioglycollic acid, mercaptoethanol, thiophenol, D-3-trolovol and dodecyl mercaptan; the solvent is one or more of methanol, ethyl acetate, water, ethanol, n-propanol, pentane, formic acid, acetic acid, diethyl ether, acetone, anisole, 1-propanol, 2-propanol, 1-butanol, 2-butanol, pentanol, butyl acetate, tributyl methyl ether, isopropyl acetate, dimethyl sulfoxide, ethyl formate, isobutyl acetate, methyl acetate, 2-methyl-1-propanol and propyl acetate;
1.3 Mixing solution A and solution B so that the mole ratio between HAuCl₄ and ligand is 1: (0.01-100), stirring them in an ice bath for 0.1~48h, adding 0.025~0.8M NaBH₄ water, ethanol or methanol solution, continuing to stir in an ice water bath and react for 0.1~12h. The mole ratio between NaBH₄ and ligand is 1: (0.01-100);
1.4 Using MWCO 3K-30K ultrafiltration tubes to centrifuge the reaction solution at 8000~17500 r/min by gradient for 10-100 min after the reaction ends to obtain ligand-bonded AuCs precipitate in different average particle sizes. The aperture of the filtration membranes for ultrafiltration tubes of different MWCOs directly decides the size of ligand-bonded AuCs that can pass the membranes. This step may be optionally omitted;
1.5 Dissolving the ligand-bonded AuCs precipitate in different average particle sizes obtained in step (1.4) in water, putting it in a dialysis bag and dialyzing it in water at room temperature for 1-7 days;
1.6 Freeze-drying ligand-bonded AuCs for 12~24h after dialysis to obtain a powdery or flocculant substance, i.e., ligand-bonded AuCs.

As detected, the particle size of the powdery or flocculant substance obtained by the foregoing method is smaller than 3 nm (distributed in 0.5-2.6nm in general). No obvious absorption peak at 520 nm. It is determined that the obtained powder or floc is ligand-bonded AuCs.

### 2. Preparation and characterization of AuCs bonded with different ligands

### 2.1 Preparation of L-NIBC-bonded AuCs, i.e. L-NIBC-AuCs

Taking ligand L-NIBC for example, the preparation and confirmation of AuCs bonded with ligand L-NIBC are detailed.
2.1.1 Weigh 1.00g of HAuCl₄ and dissolve it in 100mL of methanol to obtain a 0.03M solution A;
2.1.2 Weigh 0.57g of L-NIBC and dissolve it in 100mL of glacial acetic acid (acetic acid) to obtain a 0.03M solution B;
2.1.3 Measure 1mL of solution A, mix it with 0.5mL, 1mL, 2mL, 3mL, 4mL, or 5mL of solution B respectively (i.e. the mole ratio between HAuCl₄ and L-NIBC is 1:0.5, 1:1, 1:2, 1:3, 1:4, 1:5 respectively), react in an ice bath under stirring for 2h, quickly add 1 mL of freshly prepared 0.03M (prepared by weighing 11.3mg of NaBH₄ and dissolving it in 10mL of ethanol) NaBH₄ ethanol solution when the solution turns colorless from bright yellow, continue the reaction for 30 min after the solution turns dark brown, and add 10mL of acetone to terminate the reaction.

2.1.4 After the reaction, the reaction solution is subjected to gradient centrifugation to obtain L-NIBC-AuCs powder with different particle sizes. Specific method: After the reaction is completed, the reaction solution is transferred to an ultrafiltration tube with MWCO of 30K and a volume of 50 mL, and centrifuged at 10000r/min for 20min, and the retentate in the inner tube is dissolved in ultrapure water to obtain powder with a particle size of about 2.6 nm. Then, the mixed solution in the outer tube is transferred to an ultrafiltration tube with a volume of 50 mL and MWCO of 10K, and centrifuged at 13,000 r/min for 30 min. The retentate in the inner tube is dissolved in ultrapure water to obtain powder with a particle size of about 1.8 nm. Then the mixed solution in the outer tube is transferred to an ultrafiltration tube with a volume of 50 mL and MWCO of 3K, and centrifuged at 17,500r/min for 40 min. The retentate in the inner tube is dissolved in ultrapure water to obtain powder with a particle size of about 1.1 nm.

2.1.5 Precipitate the powder in three different particle sizes obtained by gradient centrifugation, remove the solvent respectively, blow the crude product dry with N₂, dissolve it in 5mL of ultrapure water, put it in a dialysis bag (MWCO is 3KDa), put the dialysis bag in 2L of ultrapure water, change water every other day, dialyze it for 7 days, freeze-dry it and keep it for future use.

### 2.2 Characterization of L-NIBC-AuCs

Characterization experiment was conducted for the powder obtained above (L-NIBC-AuCs). Meanwhile, ligand L-NIBC-modified gold nanoparticles (L-NIBC-AuNPs) are used as control. The method for preparing gold nanoparticles with ligand being L-NIBC refers to the reference (W. Yan, L. Xu, C. Xu, W. Ma, H. Kuang, L. Wang and N. A. Kotov, Journal of the American Chemical Society 2012, 134, 15114; X. Yuan, B. Zhang, Z. Luo, Q. Yao, D. T. Leong, N. Yan and J. Xie, Angewandte Chemie International Edition 2014, 53, 4623).

### 2.2.1 Observation of the morphology by transmission electron microscope (TEM)

The test powders (L-NIBC-AuCs sample and L-NIBC-AuNPs sample) were dissolved in ultrapure water to 2 mg/L as samples, and then test samples were prepared by hanging drop method. More specifically, 5 µL of the samples were dripped on an ultrathin carbon film, volatized naturally till the water drop disappeared, and then observe the morphology of the samples by JEM-2100F STEM/EDS field emission high-resolution TEM.

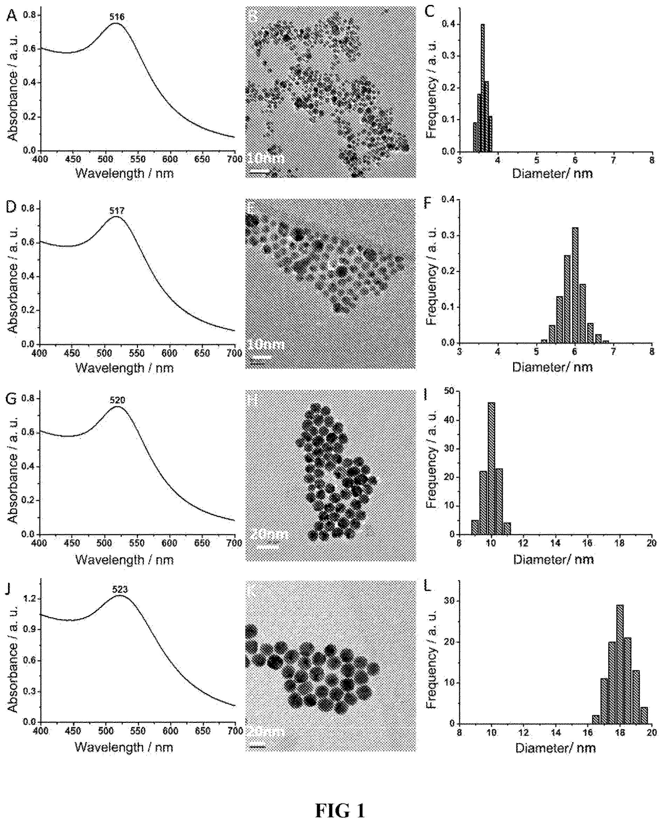

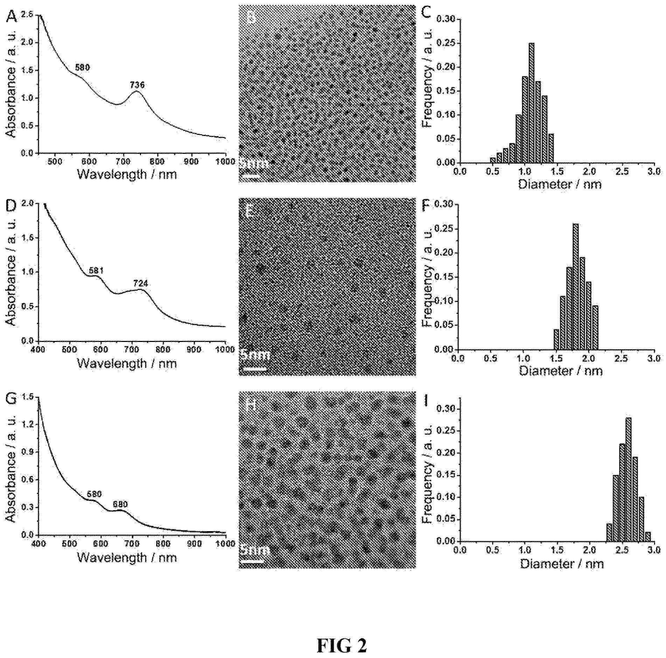

The four TEM images of L-NIBC-AuNPs are shown in panels B, E, H, and K of FIG 1; the three TEM images of L-NIBC-AuCs are shown in panels B, E, and H of FIG 2.

The images in FIG 2 indicate that each of L-NIBC-AuCs samples has a uniform particle size and good dispersibility, and the average diameter of L-NIBC-AuCs (refer to the diameter of gold core) is 1.1 nm, 1.8 nm and 2.6 nm respectively, in good accordance with the results in panels C, F and I of FIG 2. In comparison, L-NIBC-AuNPs samples have a larger particle size. Their average diameter (refer to the diameter of gold core) is 3.6 nm, 6.0 nm, 10.1 nm and 18.2 nm respectively, in good accordance with the results in panels C, F, I and L of FIG 1.

### 2.2.2 Ultraviolet (UV)-visible (vis) absorption spectra

The test powders (L-NIBC-AuCs sample and L-NIBC-AuNPs sample) were dissolved in ultrapure water till the concentration was 10mg·L⁻¹, and the UV-vis absorption spectra were measured at room temperature. The scanning range was 190-1100 nm, the sample cell was a standard quartz cuvette with an optical path of 1 cm, and the reference cell was filled with ultrapure water.

The UV-vis absorption spectra of the four L-NIBC-AuNPs samples with different sizes are shown in panels A, D, G and J of FIG 1, and the statistical distribution of particle size is shown in panels C, F, I and L of FIG 1; the UV-vis absorption spectra of three L-NIBC-AuCs samples with different sizes are shown in panels A, D and G of FIG 2, and the statistical distribution of particle size is shown in panels C, F and I of FIG 2.

FIG 1 indicates that due to the surface plasmon effect, L-NIBC-AuNPs had an absorption peak at about 520 nm. The position of the absorption peak is relevant with particle size. When the particle size is 3.6 nm, the UV absorption peak appears at 516 nm; when the particle size is 6.0 nm, the UV absorption peak appears at 517 nm; when the particle size is 10.1 nm, the UV absorption peak appears at 520 nm, and when the particle size is 18.2 nm, the absorption peak appears at 523 nm. None of the four samples has any absorption peak above 560 nm.

FIG 2 indicates that in the UV absorption spectra of three L-NIBC-AuCs samples with different particle sizes, the surface plasmon effect absorption peak at 520 nm disappeared, and two obvious absorption peaks appeared above 560 nm and the positions of the absorption peaks varied slightly with the particle sizes of AuCs. This is because AuCs exhibit molecule-like properties due to the collapse of the face-centered cubic structure, which leads to the discontinuity of the density of states of AuCs, the energy level splitting, the disappearance of plasmon resonance effect and the appearance of a new absorption peak in the long-wave direction. It could be concluded that the three powder samples in different particle sizes obtained above are all ligand-bonded AuCs.

### 2.2.3 Fourier transform infrared spectroscopy

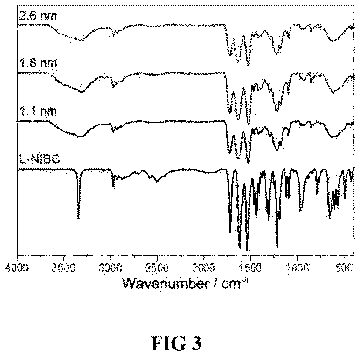

Infrared spectra were measured on a VERTEX80V Fourier transform infrared spectrometer manufactured by Bruker in a solid powder high vacuum total reflection mode. The scanning range is 4000-400 cm⁻¹ and the number of scans is 64. Taking L-NIBC-AuCs samples for example, the test samples were L-NIBC-AuCs dry powder with three different particle sizes and the control sample was pure L-NIBC powder. The results are shown in FIG 3.

FIG 3 shows the infrared spectrum of L-NIBC-AuCs with different particle sizes. Compared with pure L-NIBC (the curve at the bottom), the S-H stretching vibrations of L-NIBC-AuCs with different particle sizes all disappeared completely at 2500-2600 cm⁻¹, while other characteristic peaks of L-NIBC were still observed, proving that L-NIBC molecules were successfully bonded to the surface of AuCs via Au-S bond. The figure also shows that the infrared spectrum of the ligand-bonded AuCs is irrelevant with its size.

AuCs bonded with other ligands were prepared by a method similar to the above method, except that the solvent of solution B, the feed ratio between HAuCl₄ and ligand, the reaction time and the amount of NaBH₄ added were slightly adjusted. For example: when L-cysteine, D-cysteine, N-isobutyryl-L-cysteine (L-NIBC) or N-isobutyryl-D-cysteine (D-NIBC) is used as the ligand, acetic acid is selected as the solvent; when dipeptide CR, dipeptide RC or 1-[(2S)-2-methyl-3-thiol-1-oxopropyl]-L-proline is used as the ligand, water is selected as the solvent, and so on and so forth; other steps are similar, so no further details are provided herein.

The present disclosure prepared and obtained a series of ligand-bonded AuCs by the foregoing method. The ligands and the parameters of the preparation process are shown in Table 1.

**Table 1. Preparation parameters of AuCs bonded with different ligands in the present disclosure**

| | Ligand | Parameter | | | | |
|---|---|---|---|---|---|---|
| | | Solvent used for solution B | Feed ratio between HAuCl₄ and ligand | Time of reaction in an ice bath under stirring before addition of NaBH₄ | Mole ratio between HAuCl₄ and NaBH₄ | Time of reaction in an ice bath under stirring after addition of NaBH₄ |
| 1 | L-cysteine | Acetic acid | 1:3 | 2h | 1:2 | 0.5h |
| 2 | D-cysteine | Acetic acid | 1:3 | 2h | 1:2 | 0.5h |
| 3 | N-acetyl-L-cystein e | Ethanol | 1:4 | 1h | 1:1 | 0.5h |
| 4 | N-acetyl-D-cystein e | Ethanol | 1:4 | 1h | 1:1 | 0.5h |
| 5 | L-NIBC | Water | 1:4 | 0.5h | 1:2 | 0.5h |
| 6 | D-NIBC | Water | 1:4 | 0.5h | 1:2 | 0.5h |
| 7 | Other cysteine derivatives | Soluble solvent | 1:(0.1∼100) | 0.5h∼24h | 1: (0.1∼100) | 0.1∼24h |
| 8 | CR | Water | 1:4 | 22h | 2:1 | 0.5h |
| 9 | RC | Water | 1:4 | 20h | 2:1 | 0.5h |
| 10 | HC | Water | 1:3 | 12h | 1:2 | 2h |
| 11 | CH | Ethanol | 1:4 | 16h | 1:3 | 3h |
| 12 | GSH | Water | 1:2 | 12h | 1:1 | 3h |
| 13 | KCP | Water | 1:3 | 15h | 1:2 | 1h |
| 14 | PCR | Water | 1:4 | 16h | 1:3 | 2h |
| 15 | GSCR | Water | 1:4 | 16h | 1:3 | 1.5h |
| 16 | GCSR | Water | 1:3 | 12h | 1:2 | 2h |
| 17 | Other oligopeptides containing cysteine | Soluble solvent | 1:(0.1∼100) | 0.5h∼24h | 1:(0.1∼ 100) | 0.1∼24h |
| 18 | 1-[(2S)-2-methyl-3 -thiol-1-oxopropyl]-L-proline | Water | 1:8 | 2h | 1:7 | 1h |
| 19 | Mercaptoethanol | Ethanol | 1:2 | 2h | 1:1 | 1h |
| 20 | Thioglycollic acid | Acetic acid | 1:2 | 2h | 1:1 | 1h |
| 21 | Thiophenol | Ethanol | 1:5 | 5h | 1:1 | 1h |
| 22 | D-3-trolovol | Water | 1:2 | 2h | 1:1 | 1h |
| 23 | N-(2-mercaptoprop ionyl)-glycine | Water | 1:2 | 2h | 1:1 | 1h |
| 24 | Dodecyl mercaptan | Methanol | 1:5 | 5h | 1:1 | 1h |
| 25 | Other compounds containing thiol | Soluble solvent | 1:(0.01∼ 100) | 0.5h∼24h | 1:(0.1∼ 100) | 0.1∼24h |

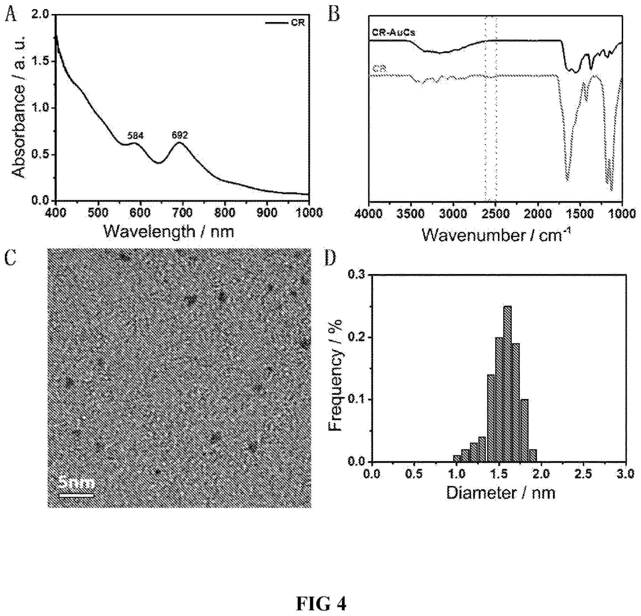

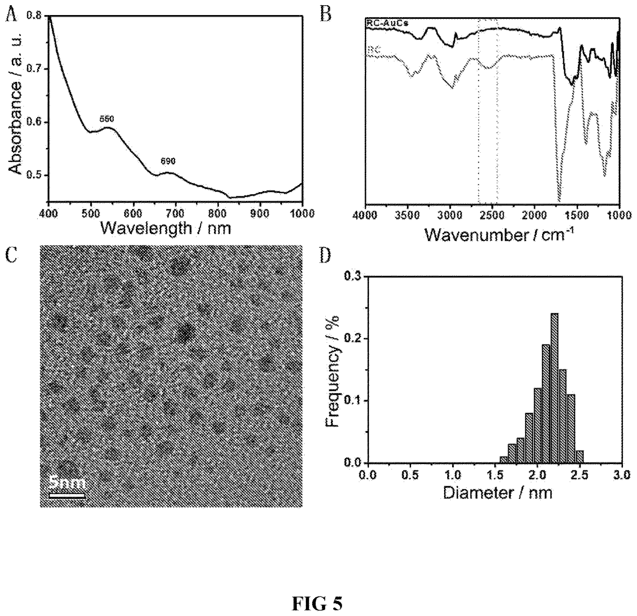

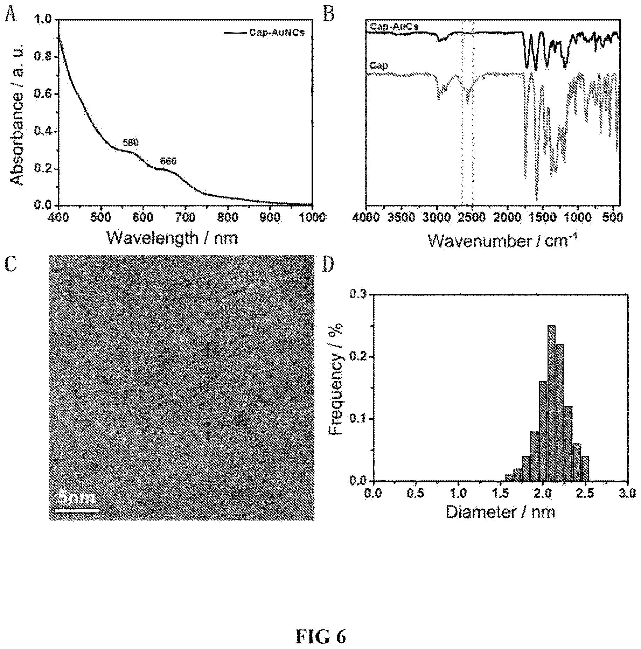

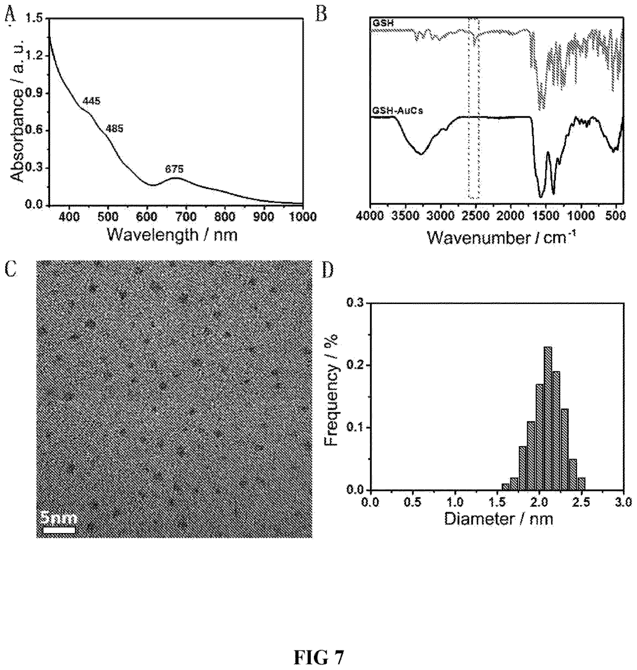

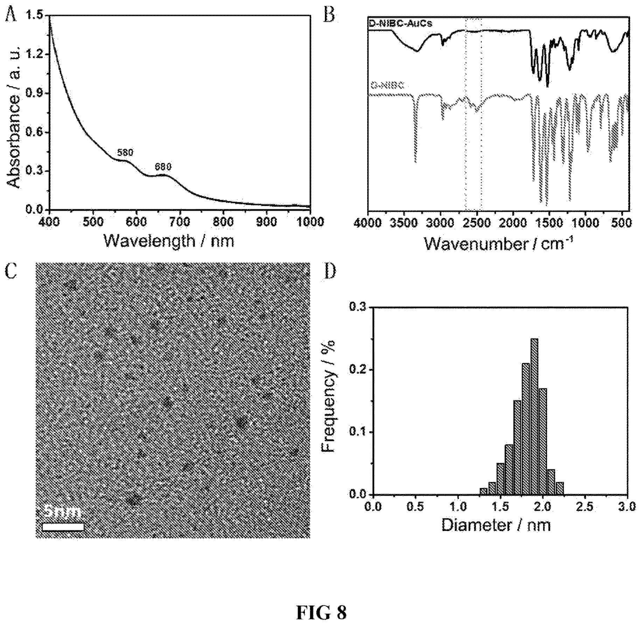

The samples listed in Table 1 are confirmed by the foregoing methods. The characteristics of five different ligand-bonded AuCs are shown in FIG 4 (CR-AuCs), in FIG 5 (RC-AuCs), in FIG 6 (Cap-AuCs) (Cap denotes 1-[(2S)-2-methyl-3-thiol-1-oxopropyl]-L-proline), in FIG 7 (GSH-AuCs), and in FIG 8 (D-NIBC-AuCs). FIGS 4-FIG 8 show UV spectra (panel A), infrared spectra (panel B), TEM images (panel C), and particle size distribution (panel D).

The results indicate that the diameters of AuCs bonded with different ligands obtained from Table 1 are all smaller than 3 nm. Ultraviolet spectra also show disappearance of peak at 520±20 nm, and appearance of absorption peak in other positions. The position of the absorption peak could vary with ligands and particle sizes as well as structures. In certain situations, there is no special absorption peak, mainly due to the formation of AuCs mixtures with different particles sizes and structures or certain special AuCs that moves the position of absorption peak beyond the range of UV-vis spectrum. Meanwhile, Fourier transform infrared spectra also show the disappearance of ligand thiol infrared absorption peak (between the dotted lines in panel B of FIGS 4-8), while other infrared characteristic peaks are all retained, suggesting that all ligand molecules have been successfully bonded to gold atoms to form ligand-bonded AuCs, and the present disclosure has successfully obtained AuCs bonded with the ligands listed in Table 1.

### 3. Animal studies

### 3.1 Materials and animals

### 3.1.1 Testing Sample

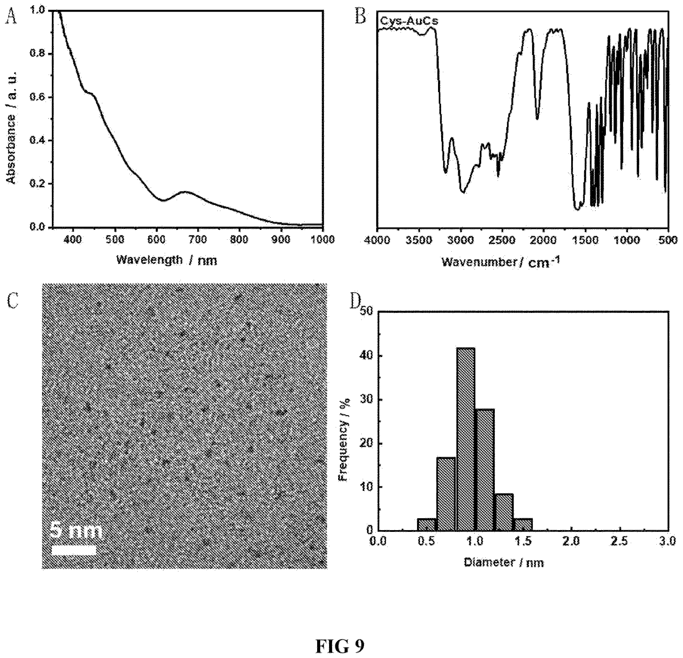

L-Cys-AuCs, the diameter of gold cores in the range of 0.5-1.5 nm (1.0 ± 0.5 nm), were used as the testing sample; the preparation method followed the above described method with slight modification. FIG 9 shows UV, infrared, TEM, and particle size distribution diagrams of ligand L-cysteine-bonded gold clusters (L-Cys-AuCs).

### 3.1.2 Positive control sample

Prednisone (Shanghai Yuanye Biotechnology Co.,Ltd).

### 3.1.3 Dose formulation

Prednisone was weighed required amount of prednisone and added proper volume of saline, gently vortexed the formulation to ensure proper mixing. L-Cys-AuCs was weighed, added with proper volume of saline, and vortexed to fully mix the suspension. All compounds were fresh prepared daily.

### 3.1.4 Animals for experiments

C57BL/6N female mice, 7-9 weeks old, were used. Animals were housed and maintained in compliance with the regulations.

**3.1.5 Experiment groups and dosages (Table 2)**

| **Group** | **Model** | **Treatment** | **Dose (mg/kg)** | **Dose volume (mL/kg)** | **Route** | **n** |
|---|---|---|---|---|---|---|
| 1 | Naïve | Saline | - | 10 | i.p. | 10 |
| 2 | EAE | Saline | - | 10 | i.p. | 10 |
| 3 | EAE | Prednisone | 10 | 10 | p.o. | 10 |
| 4 | EAE | L-Cys-AuCs | 50 | 10 | i.p. | 10 |
| 5 | EAE | L-Cys-AuCs | 20 | 10 | i.p. | 10 |
| 6 | EAE | L-Cys-AuCs | 5 | 10 | i.p. | 10 |

| | | | | | | |
|---|---|---|---|---|---|---|
| Note: EAE for experimental autoimmune encephalomyelitis; p.o. for *per os*; i.p. for intraperitoneal. | | | | | | |

### 3.1.6 Procedures

On Day 0, mice were grouped randomly by body weight, and then injected subcutaneously with 200 µg of myelin oligodendrocyte glycoprotein (MOG) peptide in complete Freund's adjuvant (CFA) into the right and left flank, 100 µl per site. Pertussis toxin (PTX) injected by i.p at 0 and 48 hours after MOG immunization. Compounds were given from day 1 to day 28 according to Table 2. The animals (n=10) were scored daily for clinical signs of EAE. At the end of the experiments, spinal cords were used for HE staining (n=5), and analysis of TNF-a, IL-17, IFN-γ by Elisa kit.

### 3.2 Results

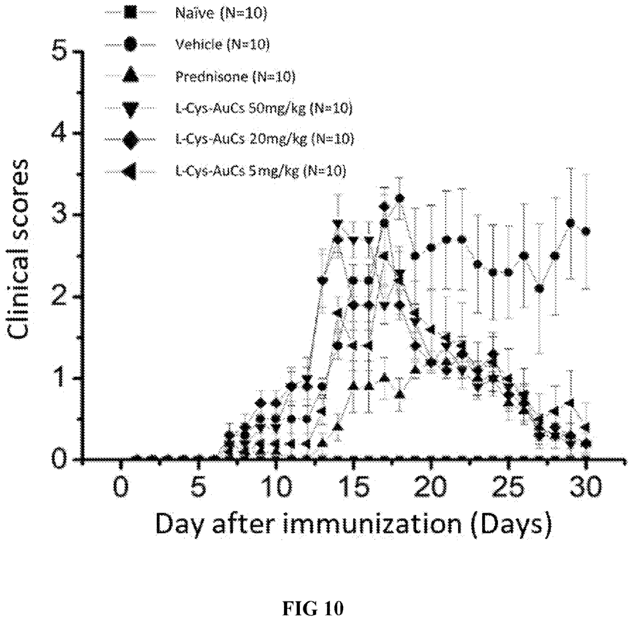

The animals were scored daily for clinical signs of EAE according to the Clinical scoring rubric for EAE (Table 3). As shown in FIG 10, L-Cys-AuCs at 50mg/kg (50mpK), 20mg/kg (20mpK) and 5mg/kg (SmpK) prevented the paralysis of EAE mice from day 17th; prednisone is a positive control, it significantly reduced clinical signs of EAE from day 13th.

**Table 1. Clinical scoring rubric for EAE**

| Score | Clinical findings |
|---|---|
| 1 | Loss of tail tonicity |
| 2 | Mild hind limb weakness |
| 3 | Partial hind limb paralysis |
| 4 | Complete hind limb paralysis |
| 5 | Complete hind limb paralysis with forelimb weakness or moribund |

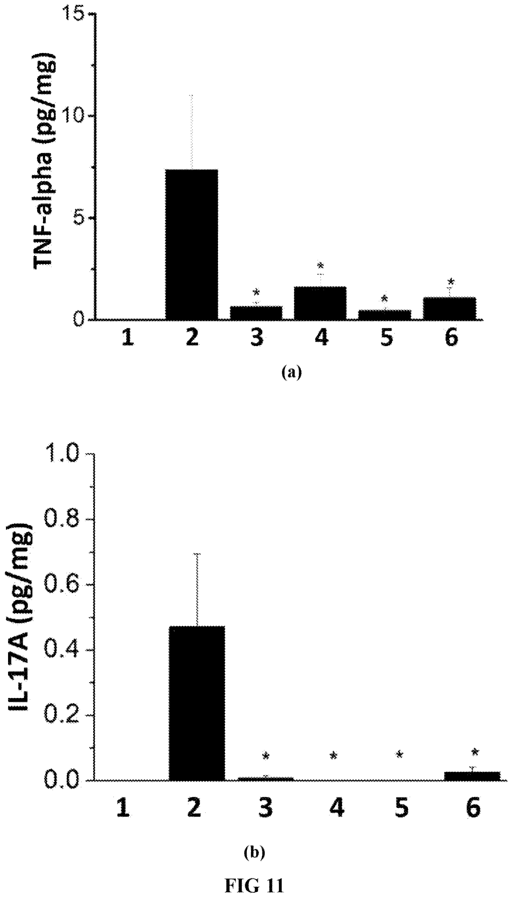

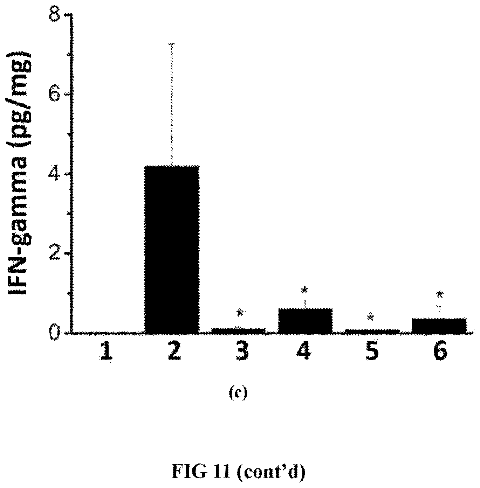

TNF-α, IL-17 and IFN-γ analysis for spinal cord by ELISA kit. TNF-α, IL-17 and IFN-γ are indicators for inflammation. L-Cys-AuCs at 50mg/kg, 20mg/kg and 5mg/kg significantly reduced the production of these inflammatory factors in EAE mice (FIG 11).

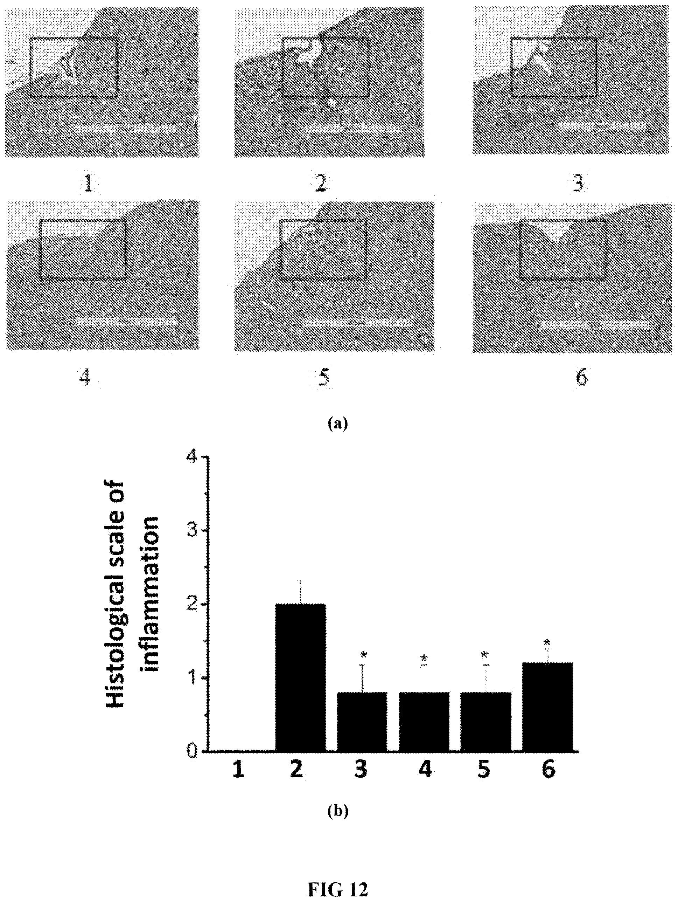

HE staining for spinal cord was used to study the infiltration of immune cells into the spinal cord. During induction of EAE, mononuclear inflammatory cells infiltrate the spinal cord, contributing to the paralysis of EAE mice, HE staining reveal a significant increase in inflammatory cells in EAE mice, and L-Cys-AuCs at 50mg/kg, 20mg/kg and 5mg/kg reduced immune cell infiltration (FIG 12(a)) and showed significantly lower histological scales of inflammation (FIG 12(b)).

Other sized L-Cys-AuCs and other ligand-bonded AuCs with different sizes also have the effects on inhibiting EAE, while their effects vary to certain extents. They would not be described in detail here.

### Industrial Applicability

Ligand-bonded AuCs can be used for the treatment of multiple sclerosis. They are suitable for industrial applications.

## Claims

1. A gold cluster (AuC) for use in the treatment of multiple sclerosis, wherein said AuC comprises:
a gold core; and
a ligand bonded to the gold core;
wherein, the ligand is one selected from the group consisting of L-cysteine and its derivatives, D-cysteine and its derivatives, and cysteine-containing oligopeptides and their derivatives;
wherein the L-cysteine and its derivatives are selected from the group consisting of L-cysteine, N-isobutyryl-L-cysteine (L-NIBC), and N-acetyl-L-cysteine (L-NAC), and wherein the D-cysteine and its derivatives are selected from the group consisting of D-cysteine, N-isobutyryl-D-cysteine (D-NIBC), and N-acetyl-D-cysteine (D-NAC);
wherein the cysteine-containing oligopeptides and their derivatives are cysteine-containing dipeptides, cysteine-containing tripeptides or cysteine-containing tetrapeptides;
wherein the cysteine-containing dipeptides are selected from the group consisting of L-cysteine-L-arginine dipeptide (CR), L-arginine-L-cysteine dipeptide (RC), L-histidine-L-cysteine dipeptide (HC), and L-cysteine-L-histidine dipeptide (CH);
wherein the cysteine-containing tripeptides are selected from the group consisting of glycine-L-cysteine-L-arginine tripeptide (GCR), L-proline-L-cysteine-L-arginine tripeptide (PCR), L-lysine-L-cysteine-L-proline tripeptide (KCP), and L-glutathione (GSH);
wherein the cysteine-containing tetrapeptides are selected from the group consisting of glycine-L-serine-L-cysteine-L-arginine tetrapeptide (GSCR), and glycine-L-cysteine-L-serine-L-arginine tetrapeptide (GCSR).

2. The AuC for use according to claim 1, wherein the gold core has a diameter smaller than 3 nm.

3. The AuC for use according to claim 1, wherein the gold core has a diameter in the range of 0.5-2.6 nm.

## Patentansprüche

1. Ein Goldcluster (AuC) zur Verwendung bei der Behandlung von Multipler Sklerose, wobei dieser AuC umfasst: einen Goldkern; und einen Liganden, der an den Goldkern gebunden ist; wobei der Ligand aus der Gruppe ausgewählt ist, die aus L-Cystein und seinen Derivaten, D-Cystein und seinen Derivaten sowie cysteinhaltigen Oligopeptiden und deren Derivaten besteht; wobei die L-Cystein und seine Derivate aus der Gruppe ausgewählt sind, die aus L-Cystein, N-Isobutyryl-L-Cystein (L-NIBC) und N-Acetyl-L-Cystein (L-NAC) bestehen, und wobei die D-Cystein und seine Derivate aus der Gruppe ausgewählt sind, die aus D-Cystein, N-Isobutyryl-D-Cystein (D-NIBC) und N-Acetyl-D-Cystein (D-NAC) bestehen; wobei die cysteinhaltigen Oligopeptide und deren Derivate cysteinhaltige Dipeptide, cysteinhaltige Tripeptide oder cysteinhaltige Tetrapeptide sind; wobei die cysteinhaltigen Dipeptide aus der Gruppe ausgewählt sind, die aus L-Cystein-L-Arginin Dipeptid (CR), L-Arginin-L-Cystein Dipeptid (RC), L-Histidin-L-Cystein Dipeptid (HC) und L-Cystein-L-Histidin Dipeptid (CH) bestehen; wobei die cysteinhaltigen Tripeptide aus der Gruppe ausgewählt sind, die aus Glycin-L-Cystein-L-Arginin Tripeptid (GCR), L-Prolin-L-Cystein-L-Arginin Tripeptid (PCR), L-Lysin-L-Cystein-L-Prolin Tripeptid (KCP) und L-Glutathion (GSH) bestehen; wobei die cysteinhaltigen Tetrapeptide aus der Gruppe ausgewählt sind, die aus Glycin-L-Serin-L-Cystein-L-Arginin Tetrapeptid (GSCR) und Glycin-L-Cystein-L-Serin-L-Arginin Tetrapeptid (GCSR) bestehen.

2. Der AuC zur Verwendung gemäß Anspruch 1, wobei der Goldkern einen Durchmesser von weniger als 3 nm hat.

3. Der AuC zur Verwendung gemäß Anspruch 1, wobei der Goldkern einen Durchmesser im Bereich von 0,5-2,6 nm hat.

## Revendications

1. Un cluster d'or (AuC) pour utilisation dans le traitement de la sclérose en plaques, où ledit AuC comprend : >
un noyau d'or ;
et un ligand lié au noyau d'or ;
où le ligand est choisi parmi le groupe constitué de L-cystéine et ses dérivés, D-cystéine et ses dérivés, et des oligopeptides contenant de la cystéine et leurs dérivés ; où la L-cystéine et ses dérivés sont choisis parmi le groupe constitué de L-cystéine, N-isobutyryl-L-cystéine (L-NIBC), et N-acétyl-L-cystéine (L-NAC), et où la D-cystéine et ses dérivés sont choisis parmi le groupe constitué de D-cystéine, N-isobutyryl-D-cystéine (D-NIBC), et N-acétyl-D-cystéine (D-NAC) ;
où les oligopeptides contenant de la cystéine et leurs dérivés sont des dipeptides contenant de la cystéine, des tripeptides contenant de la cystéine ou des tétrapeptides contenant de la cystéine ; où les dipeptides contenant de la cystéine sont choisis parmi le groupe constitué de L-cystéine-L-arginine dipeptide (CR), L-arginine-L-cystéine dipeptide (RC), L-histidine-L-cystéine dipeptide (HC), et L-cystéine-L-histidine dipeptide (CH) ;
où les tripeptides contenant de la cystéine sont choisis parmi le groupe constitué de glycine-L-cystéine-L-arginine tripeptide (GCR), L-proline-L-cystéine-L-arginine tripeptide (PCR), L-lysine-L-cystéine-L-proline tripeptide (KCP), et L-glutathion (GSH) ; où les tétrapeptides contenant de la cystéine sont choisis parmi le groupe constitué de glycine-L-sérine-L-cystéine-L-arginine tétrapeptide (GSCR), et glycine-L-cystéine-L-sérine-L-arginine tétrapeptide (GCSR).

2. Le AuC pour utilisation selon la revendication 1, où le noyau d'or a un diamètre inférieur à 3 nm.

3. Le AuC pour utilisation selon la revendication 1, où le noyau d'or a un diamètre dans la plage de 0,5-2,6 nm.
